# EUROPEAN PATENT SPECIFICATION

(11) **EP 0 429 438 B1**
(45) Date of publication and mention of the grant of the patent: **28.08.1996**
(21) Application number: 91200300.1
(22) Date of filing: 03.07.1985
(51) Int. Cl.: A61L 27/00, C08H 1/06

(54) **Bone repair using collagen**
Kollagenknochenersatzmaterial
Matériau à base de collagène pour la réparation des os

(30) Priority: 06.07.1984 US 628328; 06.07.1984 US 628335; 06.07.1984 US 628404; 06.07.1984 US 628409
(43) Date of publication of application: 29.05.1991
(62) Divisional of application: 85304757.9
(73) Proprietor: COLLAGEN CORPORATION, Palo Alto, California 94303 (US)
(72) Inventor: Wallace, Donald G., Menlo Park, California 94025 (US); Piez, Karl A., Menlo Park, California 94025 (US); Smestad, Thomas L., Palo Alto, California 94301 (US); Armstrong, Rosa, Palo Alto, California 94303 (US); Seyedin, Saeid, Saratoga California 95070 (US); McPherson, John M., Framington Massachusetts 01701 (US)
(74) Representative: Harrison, David Christopher

(56) References cited:
- EP-A- 0 052 288
- FR-A- 2 328 786
- US-A- 4 233 360
- US-A- 4 294 241
- US-A- 4 440 750

## Description

This invention relates to the field of bone repair in vertebrates, especially mammals and humans. More specifically, the invention relates to a method of repairing bone which utilizes a collagen-based implant material to facilitate repair by bone conduction. This collagen material is highly purified and non-immunogenic, and may, if desired, be xenogeneic. The collagen is a reconstituted material from skin or a bone derived material or mixture thereof.

The problem of effecting repair of defective bones has plagued mankind for centuries. Until relatively recently, the only practical course was to immobilize broken bones and to rely on nature to effect regrowth of skeletal tissue into an injury. Only with the advent of the possibility of surgery has it been possible to utilize implanted bone substitutes, not only to replace injured or diseased bone structures, but also to repair congenital or degenerative defects in the skeletal structure.

A wide range of materials have since been utilized, and elaborate designs have been disclosed for replacements of entire portions of bones, for example hip joints (US-A-3,820,167) and teeth (US-A-4,186,486). Materials employed have included metals such as titanium (EP-A-0071242, US-A-3,918,100), ceramics such as aluminum oxide (US-A-3,919,723), shaped and treated bone (US-A-3,318,774), and various bone preparations such as, for example, bone dust compacted into flexible mats (US-A-2,621,145).

It has long been understood that skeletal structures have inorganic and organic components. The mineral component which, presumably, accounts for the strength and rigidity of bone structure is predominantly a form of calcium phosphate, hydroxyapatite. The organic component is chiefly composed of a single type of protein, collagen, which serves to impart a measure of resilience thus preventing the structures from being unduly brittle. As skeletal tissue is alive, of course, additional metabolically active organic components must be included in the structure, and it is these bone cells and their active metabolites which are responsible for the naturally occurring healing and maintenance processes.

However, since the major components of bone from a quantitative standpoint are collagen and ceramic, various reconstituted implant preparations involving mixtures of similar or different ceramic materials and various types of collagen preparations have also been employed. For example, see US-A-3,443,261, Hayashi, K., et al, Arc Orthop Traumat Surg (1982) 99:265; and US-A-4,314,380).

It has been determined that bone tissue repair occurs by one of two alternative mechanisms, or a combination of both. In conductive repair, cells which are already committed to their character as bone cells (osteoprogenitor cells) move into the space of the defect from adjacent bone, and form bone directly. No special factors (other than non-specific nutrients) are required. In induction, however, this process is preceded by conversion of previously uncommitted multipotential cells into osteoprogenitor cells which first form cartilage that calcifies and degenerates and is replaced by bone. In order to acquire the capacity to do this, specific protein factors are required. The nature of these factors is not at present understood. For either conductive or inductive repair, it is required that the living tissue of the host provides the ultimate skeletal structure. Thus the implant which mediates these processes serves not as a substitute for the defective or removed bone, but rather as a matrix support for active replacement of the missing tissue.

Accordingly, attempts have been made to devise implants for defective skeletal tissue or lesions in bones and teeth which are intended precisely for this purpose. These implants do not attempt to mimic the composition of the missing bone, but rather to serve as a structural support and a guiding matrix for encroaching bone deposits derived ultimately from the adjacent fresh bone. These supports may provide only matrix support functions, i.e., mediate conductive repair, or may, in addition, include factors which stimulate the differentiation of uncommitted cells to osteoprogenitor cells by providing what are currently known as "osteogenesis factors" (OF) or "bone morphogenic proteins" (BMP). Because collagen is already a familiar material to the metabolically viable cells associated with bone growth, attempts have been made to use implants which are composed predominantly of collagen for both inductive and conductive bone repair.

For implants useful in inductive repair, US-A-4,294,753 discloses a process for preparing a bone morphogenic protein. A purification procedure for an OF which is probably not identical to the BMP of the foregoing patent is disclosed in US-A-4,434,094. These factors reside naturally in bone, and preparations of demineralized bone particles, which are used in construction of implants, presumably release this factor in operable form. Both purified and unpurified forms of these factors have been used in various implants.

Other workers have disclosed the results of attempts to utilize collagen preparations alone as a matrix for conductive bone repair activities, i.e., preparations which do not contain factors for maturation of progenitor cells into osteogenic cells, and thus mediate conductive repair.

Krekeler, V. G., et al, J Oral Surg (1981) 10:Suppl. 1:151 compared the utility of autologous spongiosa, a preparation of collagen (Collagenfleece®, Pentapharm) and binding gelatin as fillings for peridontal defects in beagles. The preparations were simply packed into the bony cavities artificially created, and the healing processes followed by polychrome sequential labeling. Collagenfleece® was found to mediate the healing, but was less effective than the autologous spongiosa transplants.

The Collagenfleece® used in the preceding preparation is derived according to a procedure disclosed in US-A-4,066,083 from pigskin. The skin is finely divided, degreased using detergent, washed, and digested with pepsin to give a viscous suspension, and the collagen precipitated by addition of saturated salt solution. The precipitate is suspended in acid, reprecipitated as a fibrous white precipitate in salt solution, washed as many times as desired, and desalted by washing with alcohol. The purified collagen is suspended in acid solution and freeze dried. It is sterilized by γ-irradiation, which may degrade or cross link the preparation. This preparation is available commercially and has been used in a number of other bone repair studies.

Joos, U., et al, Biomaterials (1980) 1:23-26, utilized Collagenfleece® as an implant in artificially damaged rabbit mandibles and found that after 2 weeks, the defects were filled with cancellous bone particles and showed complete ossification after 4 weeks. Zetzmann, D., et al, Schweiz Mschr Sabnhelik (1982) 92:119 also achieved bone regeneration in facial surgery upon use of Collagenfleece® as an implant. Springorum, H. W., et al, Z Orthop (1977) 115:686 obtained similar results using Collagenfleece® in a cortical layer defect.

Jaffee, A., et al, Archs Oral Biol (1978) 23:415; ibid. (1982) 27:999 reported successful anchoring of acrylic tooth implants in dogs using collagen solutions which were prepared from dog skin by extraction with acetic acid and trichloroacetic acid/ethanol purification. The successful anchoring of the implants was intact after a year.

Cucin, R. L., et al, New York State Journal of Medicine (1979) 1856 used atelopeptide collagen from calf skin, which had been gamma irradiated, for rib repair in rabbits and, when supported by gelatin sponge material or with autologous bone dust, to repair skull holes in dogs.

A preparation of collagen, presumably still containing the telopeptides, and cross-linked by gamma irradiation was employed in filling tooth pulp cavities and as an under the skin "bone replacement" implant as disclosed in, respectively, EP-A-0012443 and EP-A-0012959. Another cross-linked preparation is seen in EP-A-0 052 288 (=US-A-4 597 762).

None of the foregoing collagen repair procedures are completely successful. Either inflammation occurs, particularly where xenogeneic collagen is used, or healing is unsatisfactory. The present invention provides an implantable collagen preparation which is capable of conducting the ingrowing bone repair tissue from dedicated bone cells into the defect whose repair is desired. Because xenogeneic collagen can be used, large amounts are obtainable and the method can be widely applied. In addition, the invention provides bone repair compositions which offer great versatility in being adaptable to a wide range of stress-bearing requirements.

The present invention provides a composition for repairing bone defects or reconstructing the skeletal matrix of a mammal, in particular a human, by implanting in the defect purified, non-immunogenic collagen which is derived, if desired, from a species other than that being repaired. Thus, the invention provides for mediating the subject organism's natural mechanisms for bone defect repair by using a collagen preparation of general applicability which is highly purified, and which is successful in providing a matrix for new bone growth.

Fresh bone containing living osteoprogenitor cells is exposed to a bone defect and placed into contact with a preparation of collagen which is a composition derived from skin.

The skin-derived collagen is chiefly Type I collagen, includes a small amount of Type III and is typically obtained from calf skin. This type of collagen is commercially available under the trademark Zyderm® collagen soft tissue implant (ZCI) for use in removing wrinkles. The collagen preparation typified by ZCI is a reconstituted fibrillar form of atelopeptide collagen.

As is described for example in US-A-3949073, the skin-derived collagen is typically obtained from skin in a process wherein it has been dissociated into solubilized form, sterilized by filtration, and then reconstituted into fibrillar form after removal of the atelopeptides.

By varying the ratio of BCP to ZCI in a mixture of these peptides, the physical properties of the repair material can be adjusted to conform to the particular demands of the environment of the defect.

Thus in one aspect, the invention relates to a method of preparing a composition for repairing bone defects which method comprises preparing a suspension of purified reconstituted atelopeptide fibrillar skin collagen which is substantially free of cross linking, and lyophilizing the suspension. The collagen is preferably derived from calf skin.

Such a lyophilized preparation (lyophilized collagen gel or LCG) has favourable handling properties and can be extruded rolled or cast into sheets which are easily manipulated into implants. Alternatively it may be formed as a mat.

### Modes of Carrying Out the Invention

### A. Definitions

As used herein, "conductive" repair of bone defects refers to a process for replacing lost bone or for growing desired new bone, which involves the metabolism of previously committed osteoprogenitor cells. These cells are capable of producing cartilage and/or bone without induction by protein factors generally known as osteogenic or morphogenic. The process includes mechanisms whereby osteogenesis is directly effected by the committed cells, but not those wherein there is a requirement for added protein factors to produce committed cells.

"Consisting essentially of" means that the composition so defined derives all or substantially all of its relevant properties from the ingredient(s) as qualified.

It is realized that conversion of undifferentiated cells to osteoprogenitor cells may still be effected by indigenous proteins. However, as herein defined, "conductive" bone repair is mediated by external supply only of supporting matrix, and does not provide an external source of living tissue or of non-indigenous osteogenesis factor.

"Bone defect" refers to a space in the skeletal system in which it is desired that bony tissue be deposited, whether the space is created by injury to the subject, or by a malformation or degeneration of the subject's skeletal system. Such defects may be the result of simple bone breakage, decay of bone tissue because of disease, surgical removal of diseased bone tissue or of unwanted malformations, or of reconstructive or cosmetic surgery.

"Fresh bone" refers to bone in the skeletal system of the subject organism which is in healthy condition and which is treated such as by cutting to expose living tissue. It is found that in the method of the invention contact is required with the defect area by fresh bone to provide the viable cells for regeneration of bone within the defect.

"Preparation of collagen" refers to a composition which contains as its major component some form of collagen protein.

"Xenogeneic" refers to a species which is different from that of the subject being treated.

"Free from impurities" or "purified" refers to those impurities which are normally associated with the collagen or other preparation in its natural state. Thus, collagen prepared from calf skin is free from impurities when other components of calf skin have been substantially removed; that from bone, when other components of bone are eliminated.

"Reconstituted" collagen refers to collagen which has been disassembled into individual triple helicial molecules with or without their telopeptide extensions and brought into solution, and then regrouped into "fibrillar" form. In this form, the fibrils consist of long, thin collagen molecules staggered relative to one another by multiples of about 1/4 of their length. This results in a banded structure which can be further aggregated into fibers.

"Substantially free of cross-linking" refers to collagen which has undergone removal of the telopeptides, so as to result in a preparation lacking the native capacity for cross-link formation. Such preparations remain substantially cross-link free if not deliberately cross-linked by, for example, treating with glutaraldehyde, or subjected to treatment imposing a spurious form of such linkage. For example, treatments often used for sterilizing purposes, such as high temperature and γ-irradiation have the effect of causing the formation of additional cross-links between helices. In the preparation used in the invention, the skin-derived collagen is sterilized by microfiltration while still in solution and handled under sterile conditions thereafter, thus avoiding the result of unwanted cross-link formation.

"Bone collagen powder" (BCP) refers to a purified atelopeptide preparation of collagen derived from demineralized bone. This preparation consists essentially of collagen per se, and does not contain metabolically active proteins. As it originates in bone, it is composed of Type I collagen, and it mimics the native 3-dimensional structure found in bone.

### B. Detailed Description

### B.1 Applications

The bone defects to which the invention applies include any cavity in osseous tissue whose filling is required in order to integrate the skeletal system. Medical or veterinary procedures of bone defect repair which are appropriate as vehicles for the use of the method of the invention include reconstructive surgery, removal of diseased osseous tissue and replacement with overlay or prosthesis, tightening of teeth, replacement of teeth, repair of traumatic injury, and the like. Precise means for applying the collagen preparations of the invention is dependent on the nature of the bone defect and the procedure selected to counteract it, as will be understood by those skilled in the art. However, in general, the compositions of the invention can be made into a paste or gel and molded into the defect by surgically packing the paste into the defect. The collagen preparation may also be injected into the defect when mixed so as to form a thinner suspension. In all cases, however, the defect to be treated must first be cleansed in such a way so as to expose fresh bone surface so that living bone cells are placed in communication with the defect. The fresh bone surface is required in order to provide a source of osteoprogenitor cells needed to synthesize the permanent bone structure.

### B.2 Collagen Preparations

Native collagen consists in large part of a triple helical structure containing repeating triplet sequences composed of glycine linked to two additional amino acids, commonly proline and hydroxyproline; thus, glycine appears in every third position in the chain. In addition, all collagen chains contain regions at each end which do not have the triplet glycine sequence and are thus not helical. These regions are thought to be responsible for the immunogenicity associated with most collagen preparations. Immunogenicity can, in large part, be mitigated by removal of these regions to produce "atelopeptide" collagen. This can be accomplished by digestion with proteolytic enzymes such as trypsin or pepsin. Because of differing specificities of these proteases, the degree of completeness of removal of the telopeptides varies. Thus certain proteases, which effect the most complete removal, are preferred. Included among these is trypsin, which results in removal of substantially all of the telopeptide portions.

The non-helical telopeptide regions are also required to form the cross-links which aid in stability of the fibrillar structure. These regions contain aldehydes capable of cross-linkage to lysine residues. Atelopeptide collagen must be cross-linked artificially, if it is so desired.

While all collagens share the foregoing characteristics, they have been subclassified into approximately ten types depending on the precise amino acid sequence in the individual chains of the triple helix, the carbohydrate content, and the presence or absence of disulfide cross-linking. The most common subtypes are Type I which is present in skin, tendon, and bone, and which is made by fibroblasts, and Type III which is found primarily in skin. Other types reside in specialized membranes or cartilage or at cell surfaces. Types I and III contain similar numbers of amino acids in their helices; however, Type III but not Type I contains two adjacent cysteines at the C-terminal ends of the triple helix which are capable of forming interchain cross-links.

Type I collagen contains one α2 (I) and two α1(I) chains each of which contains 1014 amino acids in its triplet region; there are several carbohydrate moieties present on each chain. Type III collagen contains only α1(III) (3 chains) which contain 1026 residues in their triplet regions. As stated above, the presence in Type III of a pair of adjacent cysteine residues at the carboxy terminal end of the triplet region results in stability of the interchain cross-links. Both collagens contain short non-triplet ends (telopeptides). The reconstituted fibrillar atelopeptide collagen used in this invention contains the atelopeptide forms of both Type I and Type III; the bone collagen powder consists of the atelopeptide form of Type I exclusively.

### The Skin-Derived Collagen

The atelopeptide reconstituted fibrillar skin collagen preparations useful in the compositions of this invention are typified by the purified calfskin-derived atelopeptide collagen reconstituted fibrillar suspensions sold commonly under the trademark ZYDERM® collagen implant (ZCI).

This and other preparations of skin-derived purified atelopeptide, reconstituted fibrillar collagen are well known in art. ZCI has been used extensively in soft tissue applications, including most prominently, the removal of wrinkles and depressed scars by injection of the preparation just under the skin. However, such preparations have not been used for bone repair except in conjunction with supporting materials which also contain OF and thus are directed to inductive bone repair.

As this collagen preparation is derived from calf skin, it contains mainly Type I collagen with approximately 1-5% Type III. The aletopeptide collagen is sterilized while still in solution by suitable filtration techniques and thus is not degraded or cross-linked. It is reconstituted into fibrillar form and packaged under sterile conditions.

### The Lyophilized Skin Collagen

When this collagen material is lyophilized to form lyophilized collagen gel (LCG), it exhibits the ability to entrench itself in the implanted or filled cavity and to resist mobilization from the desired location due to its superior structural integrity. In addition, LCG matrices exhibit the desirable and necessary property of supporting the healing and regrowth of bone tissue into the implanted area. As described in further detail below, the LCG preparations of the invention may be prepared over a range of physical properties which are controlled by the pH, freezing rate, and concentration of the suspension during lyophilization. Whatever the properties desired, the LCG preparations of the invention are partially characterized in that they consist of atelopeptide reconstituted fibrillar collagen substantially free of impurities. If the preparation has been sterilized by microfiltration and processed under sterile conditions thereafter, it is also substantially free from cross-linking. The added lyophilization process results in a mat which is of sufficient cohesiveness to allow it to be easily cut simply using scissors or sharp blade, into the appropriate shape for clinical application. Wetting the mat produces a putty-like material which can be formed into any desired shape and placed into the defect.

We and others have disclosed forms of lyophilized collagen for medical applications. These collagen preparations, however, differ from those of the present invention as do their uses. Battista, US-A-3,471,598, discloses a lyophilized form of a preparation of an intermediate microcrystalline form of collagen, which is obtained by treatment of bovine skin with hydrochloric acid to swell and separate the collagen fibers. The collagen is not purified nor are the atelopeptides removed. The material so prepared is regarded as being suitable for water absorbent sponges; clearly, the impure nature and the immunogenicity of such preparations would make them relatively undesirable for direct medical use. In addition, the acid used to prepare the intermediate form remains in the sponge. Kuntz, et al, US-A-3,368,911, disclose an alternate method of preparing absorbent collagen sponges so as to be devoid of the acid which, substitutes carbonic acid for the comparatively non-volatile acids used in Battista. While the preparation is disclosed to employ substantially pure collagen fibrils, the atelopeptides have not been removed. US-A-4,440,750 discloses a plastic dispersion of demineralized bone powder and reconstructed atelopeptide collagen. Miyata, US-A-4,294,241, discloses lyophilized collagen sheets for skin dressing. These sheets are prepared from atelopeptide collagen reconstituted into fibrils; however, they differ from the LCG of the present invention in that they are artificially cross-linked. Ries, in US-A-4,066,083, discloses the method of preparation of the commercially available product, Collagenfleece®, for wound treatment. Luck and Daniels, US-A-4,233,360, describe a lyophilized preparation which results in a foam rather than the mats within the present invention.

See also FR-A-2,328,786 (=US-A-4,140,537, GB-A-1,551,163), where twisted intertwined fibrils of an atelopeptide skin collagen, having a mean diameter of at least 500 µm, are prepared by subjecting a collagen solution to high shear during desolubilization. The product may be lyophilized.

In general, the LCG of the present invention is prepared from a purified reconstituted fibrillar atelopeptide collagen such as, for example, Zyderm® I collagen implant or Zyderm® II collagen implant, both trademarks for reconstituted collagen preparations prepared from bovine skin. Other sources of the reconstituted purified skin collagen can be used, such as, for example, the skins of other mammals. In any event, however, the starting material must be prepared from these sources by processes which result in essentially pure collagen fibrils which are reconstituted from the solubilized form. In addition, the telopeptides must have been removed by suitable processes, such as, for example, trypsin digestion. Typically, such purified preparations are prepared by finely dividing the starting material, treating it with enzymes and/or suitable conditions of pH so as to destroy or extract non-collagen materials, treating the collagen with a digestive enzyme such as trypsin to remove the telopeptides, microfiltering to sterilize, and reconstituting the collagen fibrils from suspension by appropriate adjustment of pH and salt concentration, i.e., from acidic to neutral. Such preparations are known in the art, and indeed, the results thereof are commercially available.

The use of reconstituted collagen to prepare the lyophilized form is significant also from the perspective of non-degradative sterilization processes. As the collagen is, at one point in its preparation, in solubilized form, sterilization can be conducted by microfiltration - an entirely neutral process which causes no significant chemical or physical change in the structure. If further steps are then carried out under sterile conditions, so that no further sterilization is necessary, the resultant product is neither degraded or cross-linked as would be the case if more harsh sterilization methods, such as heat or γ-irradiation were used.

In one method of preparing LCG within the invention a suspension of suitable collagen is brought to a concentration of approximately 20-100 mg/ml and cast into molds or extruded into sheets. These forms are then frozen at about -10°C to -50°C and lyophilized at approximately 10-100 millitorr with a condenser temperature at about -45°C to -55°C, and the shelf temperature at approximately -30°C. Lyophilization is carried out for 1-7 days before increasing the shelf temperature to approxiately 15°C-25°C for about 24 hours before removing the LCG from the lyophilizer.

Alternatively, a solubilized form of purified collagen can be used as a starting material. Such a solution is obtained by dissolving purified atelopeptide collagen in aqueous solution at about pH 2-3, optionally sterilizing by microfiltration, and precipitating the reconstituted form by bringing the pH to approximately neutrality with, for example, phosphate buffer. The resulting precipitated collagen is harvested by centrifugation, and a pelleted collagen resuspended in neutral buffer and treated as set forth above.

An alternative method of lyophilization which yields, for some applications, superior results, is a modified form of the foregoing procedure. The collagen suspension is cast into sterile culture plates (e.g., 35 mm), and the resulting cast is smoothed, e.g., with a spatula. The casts are then frozen and held at approximately -100°C to - 50°C, preferably around -80°C for about one hour prior to lyophilization. Lyophilization is conducted at about 10-100 millitorr over approximately 24 hours until dry to yield porous mats a few mm thick.

### C. Examples

The following will illustrate but not limit the invention.

### C.1 Preparation of ZCI

Zyderm® Collagen implant (available from Collagen Corporation, Palo Alto, CA) was used as the reconstituted fibrillar atelopeptide component. The reconstituted native type collagen fibrils were used at a concentration of about 35 mg/ml. The preparation had been sterilized during processing by filtration and thus was not subjected to degradative procedures such as heat or irradiation.

### C.2 Preparation of LCG

A. Pepsin-solubilized bovine hide collagen in aqueous solution at about pH 2-3 which was sterilized by microfiltration was adjusted to pH 7.4 with phosphate buffer. The precipitated collagen was harvested by centrifugation and then suspended in phosphate buffer pH 7.4 to 35 mg/ml. The solution was cast into sheet form, and the sheets frozen at -25°C and then lyophilized at 25 millitorr with the condenser temperature at -50°C. The lyophilized sheets were then stored at -30°C for 5 days before the temperature was again raised to 20°C for 24 hr immediately prior to use.

B. In an alternative (and preferred) method, approximately 5 cc portions of ZYDERM® Collagen Implant were cast into sterile 35 mm tissue culture plates and smoothed with a spatula. The collagen gel was frozen and held at -80°C for approximately one hour. The frozen collagen gel was then lyophilized for approximately 24 hours until dry to yield porous white disks 35 mm in diameter and 3 to 5 mm thick.

### C.3 Repair of Cranial Defects Using LCG

Rats approximately 8 weeks old were anesthesized, the scalp reflected and the cranial periosteum removed. Bilateral 3 x 7 mm full thickness defects were placed in the parietal bones of the cranium with a dental burr. The lyophilized collagen as prepared in C.2.B was cut into strips slightly larger than the defects so as to effect a tight seal between the defect and the implant. Some of the strips were placed in the defects dry and allowed to hydrate in situ while others were prehydrated prior to implantation. After implantation, the scalp was repositioned and sutured. The rate of healing within the defects was evaluated by histology at 2 and 4 weeks post-implantation.

At 2 weeks, the implants were well infiltrated with connective tissue cells and blood vessels but new bone formation was very limited. While non-implanted defects of the type described in the previous paragraph did not heal and remained filled with soft tissue, the repaired detect showed areas of new bone forming throughout the implant by 28 days. Early evidence of fusion with pre-existing bone could be seen at the interface of the cut edges of the defect and the implant. By 56 days mature bone with well-defined marrow cavities were present throughout the defect.

LCG is easily cut and manipulated to form inserts capable of supporting conductive bone growth. The lyophilized reconstituted collagen fiber mats which are obtained provide a convenient and suitable material for this purpose.

## Claims

1. A method of preparing a composition for effecting conductive repair of a bone defect in a mammal, which method comprises preparing a suspension of purified atelopeptide reconstituted fibrillar skin collagen which is substantially free of cross-linking, and lyophilizing the suspension.

2. The method according to claim 1 which includes forming the preparation into a sheet.

3. The method according to claim 1 or claim 2 wherein the suspension is prepared from calf skin.

4. A purified collagen preparation consisting essentially of a lyophilized reconstituted atelopeptide fibrillar skin collagen in the form of a sheet.

5. A purified collagen preparation according to claim 4 which is substantially free of cross-linking.

6. A process for preparing lyophilized collagen mats suitable for use as bone implants which process comprises:
(a) forming a suspension of purified atelopeptide reconstituted fibrillar skin collagen into sheets, and
(b) lyophilizing the resultant.

## Patentansprüche

1. Verfahren zur Herstellung einer Zusammensetzung zur Durchführung einer konduktiven Reparatur eines Knochendefekts in einem Säugetier, welches Verfahren das Herstellen einer Suspension von gereinigtem, rekonstituiertem, fibrillärem Atelopeptidhautkollagen, das im wesentlichen frei von Vernetzungen ist, und das Lyophilisieren der Suspension umfaßt.

2. Verfahren nach Anspruch 1, das das Formen des Präparats zu einer Bahn umfaßt.

3. Verfahren nach Anspruch 1 oder 2, worin die Suspension aus Kalbshaut hergestellt wird.

4. Gereinigtes Kollagenpräparat, das im wesentlichen aus einem lyophilisierten, rekonstituierten, fibrillären Atelopeptidhautkollagen in Bahnform besteht.

5. Gereinigtes Kollagenpräparat nach Anspruch 4, das im wesentlichen frei von Vernetzungen ist.

6. Verfahren zum Herstellen lyophilisierter Kollagenmatten, die sich zur Verwendung als Knochenimplantate eignen, welches Verfahren die folgenden Schritte umfaßt:
(a) das Formen einer Suspension von gereinigtem, rekonstituiertem, fibrillärem Atelopeptidhautkollagen zu Bahnen und
(b) Lyophilisieren des Erhaltenen.

## Revendications

1. Méthode de préparation d'une composition pour effectuer une réparation, par conduction, d'un défaut de l'os chez un mammifère, laquelle méthode consiste à préparer une suspension de collagène de peau fibrillaire reconstitué atélopeptidique purifié qui est sensiblement exempt de réticulations et à lyophiliser la suspension.

2. Méthode selon la revendication 1 qui consiste à former la préparation en une feuille.

3. Méthode selon la revendication 1 ou la revendication 2 où la suspension est préparée à partir de peau de veau.

4. Préparation de collagène purifié consistant essentiellement en un collagène de peau fibrillaire atélopeptidique reconstitué et lyophilisé sous la forme d'une feuille.

5. Préparation de collagène purifié selon la revendication 4 qui est sensiblement exempt de réticulations.

6. Procédé de préparation de tapis de collagène lyophilisé appropriés à une utilisation comme implants de l'os, lequel procédé consiste à :
(a) former une suspension de collagène de peau fibrillaire reconstitué atélopeptidique purifié en feuilles, et
(b) lyophiliser le résultat.
